# FASCICULE DE BREVET EUROPEEN

(11) **EP 0 822 250 B1**
(45) Date de publication et mention de la délivrance du brevet: **24.10.2001**
(21) Numéro de dépôt: 97401843.4
(22) Date de dépôt: 31.07.1997
(51) Int. Cl.: C12F 3/10, C07C 29/76, C07C 33/22

(54) **Procédé d'extraction du 2-phényl éthanol**
Verfahren zur Extraktion von 2-Phenylethanol
Process for the extraction of 2-phenylethanol

(30) Priorité: 01.08.1996 FR 9609730
(43) Date de publication de la demande: 04.02.1998
(73) Titulaire: PERNOD-RICARD, 75008 Paris (FR)
(72) Inventeur: Savina, Jean-Pierre, 91800 Brunoy (FR); Kohler, Danièle, 94170 Le Perreux (FR); Brunerie, Pascal, 94440 Santeny (FR)
(74) Mandataire: Le Forestier, Eric

(56) Documents cités:
- DATABASE WPI Section Ch, Week 8004 Derwent Publications Ltd., London, GB; Class B05, AN 80-06919C XP002029617 & SU 662 101 A (GEORG ORCHARD VINEY) , 15 mai 1979

## Description

L'invention a pour objet un procédé d'extraction du 2-phényl éthanol.

Le 2-phényl éthanol dont la formule développée est présentée ci-après est un composé aromatique qui, lorsqu'il est purifié, dégage une agréable odeur de rose et, à ce titre, il est très utilisé dans les industries agro-alimentaires, les cosmétiques et la parfumerie.

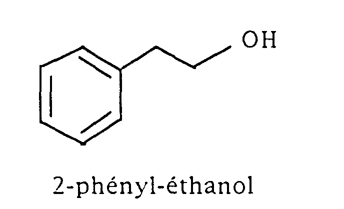

Le 2-phényl éthanol naturel peut être obtenu par distillation de pétales de roses, ce qui nécessite une mise en oeuvre relativement lourde pour la culture de roses spécifiquement à cet effet, et se répercute inévitablement sur le coût du 2-phényl éthanol naturel ainsi obtenu.

Par ailleurs, il est connu que le 2-phényl éthanol est un métabolite normal de la fermentation par des levures et qu'il se trouve à des concentrations de 10 à 35 mg/l dans tous les produits fermentés. Cependant, ces concentrations sont trop faibles pour justifier la mise au point de procédés rentables d'extraction du 2-phényl éthanol.

L'invention a pour objet un nouveau moyen d'obtention du 2-phényl éthanol naturel. Ce nouveau procédé consiste à extraire le 2-phényl éthanol des résidus de distillation obtenus lors de l'élaboration de l'alcool par distillation.

Deux techniques différentes sont traditionnellement utilisées pour l'obtention d'alcool par distillation : la distillation en continu sur colonne et la distillation discontinue en deux étapes ou technique double chauffe en alambics discontinus. Tous les résidus de distillation obtenus lors de l'application de ces techniques contiennent du 2-phényl éthanol dont la concentration est variable selon la matière première utilisée pour la fermentation ou la technique de distillation (40 à 150 mg/l).

En particulier, les résidus issus de la première distillation dans le cas d'une double distillation en alambics discontinus ou les résidus issus de distillation discontinue sont caractérisés par une concentration en 2-phényl éthanol de l'ordre de 40 à 70 mg/l. Dans ces cas, l'extraction directe de la molécule est difficile en raison de la grande quantité de matière en suspension. Néanmoins, en raison de leur forte charge polluante, les résidus sont souvent concentrés par évaporation. Les condensats générés ne contiennent pas de matière en suspension et présentent des teneurs très basses en solides dissous. La concentration en 2-phényl éthanol dans les condensats est similaire à celle des résidus avant évaporation.

Avantageusement, le procédé selon la présente invention est mis en oeuvre pour l'extraction du 2-phényl éthanol à partir de résidus de seconde distillation dans le cas d'une double distillation en alambics discontinus. Ces résidus sont appelés "Spent Lees" dans le cas de la fabrication de whisky et "Secondes de bonne chauffe" dans le cas de la fabrication du cognac. En effet, c'est dans ces résidus de deuxième distillation que la concentration en 2-phényl éthanol est la plus importante et de l'ordre de 100 à 150 mg/l en ce qui concerne les Spent Lees.

Avantageusement, l'extraction du 2-phényl éthanol à partir des résidus de deuxième distillation obtenus lors de l'élaboration de l'alcool, est précédée d'une étape de clarification desdits résidus. En effet, cette clarification facilite considérablement l'étape suivante d'extraction du 2-phényl éthanol du fait que ces résidus contiennent des matières en suspension (20 à 250 mg/l) particulièrement colmatantes en raison de leur aspect cireux.

Encore plus avantageusement, la clarification des résidus est réalisée par une microfiltration tangentielle qui présente l'avantage de débarrasser totalement les résidus des particules en suspension. De plus, cette technique s'est avérée très avantageuse d'un point de vue économique.

Dans le cadre de la présente invention, les membranes mises en oeuvre pour réaliser la microfiltration tangentielle sont des membranes minérales classiques.

Les membranes minérales présentent l'avantage de pouvoir travailler efficacement à des températures relativement élevées (50 à 60°C) ce qui permet d'obtenir des flux de perméat importants. De plus, les opérations de nettoyage s'en trouvent simplifiées.

Les membranes utilisées dans le cadre de l'invention sont des membranes TECHSEP KERASEP ™ présentant un seuil de coupure de 0,10 µ avec des canaux intérieurs de diamètre 3,5 mm. Les flux de perméat obtenus se situent entre 200 et 600 l/h/m². Cependant, l'invention n'est pas limitée à ce type de membranes.

Il est ainsi possible de clarifier les résidus de deuxième distillation avec un facteur de conversion supérieur à 90 %.

Selon un autre mode de réalisation avantageux du procédé conforme à l'invention, l'extraction du 2-phényl éthanol à partir des résidus clarifiés est réalisée par échange d'ions au moyen de résines adsorbantes. Dans le cadre de la présente invention, les résines utilisées sont des résines adsorbantes capables de fixer des composés lipophiles. Les meilleurs résultats ont été obtenus avec les résines Dowex S 112 de la société DOW, mais d'autres résines peuvent également convenir.

Le 2-phényl éthanol ainsi adsorbé sur la résine est ensuite récupéré de façon classique par élution de la colonne avec un alcool tel que l'éthanol ou un autre solvant organique.

Selon un autre mode de réalisation avantageux du procédé conforme à l'invention, l'extraction du 2-phényl éthanol à partir des résidus clarifiés est réalisée par osmose inverse. En effet, il a été constaté, de façon inattendue, que cette technique permet de façon très efficace de retenir le 2-phényl éthanol en le concentrant à l'aide de membranes d'osmose inverse.

L'osmose inverse donnant lieu à un rétentat dans lequel est retenu le 2-phényl éthanol et à un perméat consistant en tout ce qui n'a pas été retenu, le rétentat subit en fait un certain nombre de cycles d'osmose inverse, ce qui a pour effet de le concentrer un peu plus à chaque cycle par élimination de l'eau. C'est ainsi que le rétentat peut être concentré cent fois par élimination de 99 % de l'eau.

Cependant, des condensats résultant de la concentration par évaporation de résidus à partir de la première distillation dans le cas d'une double distillation en alambics discontinus ou de résidus issus d'une distillation continue peuvent être traités par le procédé selon l'invention. Néanmoins, pour atteindre une concentration en 2-phényl éthanol de 10 g/l dans le rétentat produit par osmose inverse, le facteur de concentration volumique doit être supérieur à celui correspondant au traitement des résidus de seconde distillation.

Il est à souligner que lorsque la microfiltration tangentielle est utilisée pour clarifier les résidus de deuxième distillation, les flux d'osmose inverse subséquents s'en trouvent considérablement améliorés.

Les membranes d'osmose inverse utilisables dans le cadre de la présente invention sont des membranes présentant une porosité suffisamment faible pour pouvoir retenir le 2-phényl éthanol. C'est le cas notamment des membranes d'osmose inverse classiquement utilisées pour le dessalement de l'eau. En effet, ce type de membrane étant hydrophile alors que le 2-phényl éthanol est hydrophobe, celui-ci ne passe pas à travers la membrane. A titre d'exemple de membrane, on peut citer les membranes Filmtec de la société DOW (référence BW-30-330) dont le taux de rétention du 2-phényl éthanol reste supérieur à 98 % quelles que soient les conditions de fonctionnement. Mais d'autres types de membranes d'osmose inverse peuvent également convenir.

En ce qui concerne la récupération du 2-phényl éthanol à partir du rétentat obtenu par osmose inverse, celle-ci peut être classiquement réalisée au moyen d'une extraction solvant suivie d'une purification par distillation.

L'invention sera mieux comprise au moyen des exemples suivants.

### Exemple 1: Extraction de 2-phényl éthanol sur colonne adsorbante

Un essai pilote a été réalisé sur colonne adsorbante de 50 cm de hauteur et 10 cm de diamètre contenant 4 1 de résines Dowex S 112.

250 1 de Spent Lees ont été passés sur cette colonne à un débit variant de 55 à 70 1/h.

L'élution a été réalisée avec 10 1 d'alcool à 96% à un débit de 40 1/h, l'éluat étant fractionné en fractions de 1 1 chacune. Le dosage de 2-phényl éthanol est réalisé sur chacune des fractions en chromatographie en phase liquide. Les résultats sont rassemblés dans le tableau 1.

**TABLEAU I**

| FRACTIONS | 1 | 2 | 3 | 4 | 5 | 6 | 7 | 8 | 9 |
|---|---|---|---|---|---|---|---|---|---|
| 2-phényl éthanol en g/l | 0,013 | 0,25 | 23,81 | 24,18 | 11,13 | 5,31 | 2,106 | 0,1 | 0,47 |

Les fractions 3, 4, 5, 6 et 7 ont été cumulées puis évaporées à l'évaporateur rotatif sous vide à 40°C. Environ 100 g d'une huile aromatique contenant 85 % de 2-phényl éthanol ont été obtenus. Le 2-phényl éthanol peut ensuite être purifié par rectification.

L'analyse GC/MS de l'huile brute obtenue montre la présence, à côté du 2-phényl éthanol, de nombreux acides organiques et composés soufrés.

### Exemple 2 : Extraction du 2-phényl éthanol par osmose inverse

1650 l de Spent Lees avec une concentration de l'ordre de 110 mg/l de 2-phényl éthanol ont été prélevés en fin de deuxième distillation dans un alambic industriel. Ces Spent Lees ont d'abord été traités par microfiltration tangentielle sur un pilote de microfiltration équipé de 0,25 m² de membrane Techsep Kerasep possédant un seuil de coupure de 0,10 µ.

1500 l de Spent Lees clarifiés ont été obtenus après 28 h de traitement.

Ces Spent Lees clarifiés ont ensuite été concentrés par osmose inverse sur un pilote équipé de 2,5 m² de membranes Filmtec BW 30-330.

Après 16 heures de fonctionnement, il a été obtenu 17 l d'un rétentat titrant 9,1 g/l de 2-phényl éthanol et 1483 litres d'un perméat titrant 4,1 mg/l de 2-phényl éthanol.

## Revendications

1. Procédé d'extraction de 2-phényl éthanol dans lequel le 2-phényl éthanol est extrait à partir des résidus de distillation obtenus lors de l'élaboration de l'alcool.

2. Procédé d'extraction de 2-phényl éthanol selon la revendication 1, dans lequel le 2-phényl éthanol est extrait à partir des résidus de deuxième distillation obtenus lors de l'élaboration de l'alcool par double distillation.

3. Procédé d'extraction de 2-phényl éthanol selon la revendication 1 ou 2, dans lequel l'extraction du 2-phényl éthanol est précédée d'une étape de clarification des résidus.

4. Procédé d'extraction de 2-phényl éthanol selon la revendication 3, dans lequel la clarification des résidus est réalisée par microfiltration tangentielle.

5. Procédé d'extraction de 2-phényl éthanol selon la revendication 3 ou 4, dans lequel l'extraction du phényl éthanol est réalisée au moyen d'une colonne adsorbante.

6. Procédé d'extraction de 2-phényl éthanol selon la revendication 5, dans lequel le 2-phényl éthanol est ensuite récupéré par élution de la colonne avec un alcool ou tout autre solvant organique.

7. Procédé d'extraction de 2-phényl éthanol selon la revendication 3 ou 4, dans lequel l'extraction de 2-phényl éthanol est réalisée par osmose inverse.

8. Procédé d'extraction de 2-phényl éthanol selon la revendication 7, dans lequel le 2-phényl éthanol contenu dans le rétentat obtenu par osmose inverse est ensuite récupéré au moyen d'un solvant.

## Patentansprüche

1. Verfahren zur Extraktion von 2-Phenylethanol, in dem der 2-Phenylethanol ausgehend von Destillationsrückständen, die bei der Verarbeitung von Alkohol erhalten werden, extrahiert wird.

2. Verfahren zur Extraktion von 2-Phenylethanol gemäß Anspruch 1, in dem der 2-Phenylethanol ausgehend von Rückständen der zweiten Destillation, die bei der Verarbeitung von Alkohol durch doppelte Destillation erhalten werden, extrahiert wird.

3. Verfahren zur Extraktion von 2-Phenylethanol gemäß Anspruch 1 oder 2, in dem der Extraktion des 2-Phenylethanols eine Reinigungsstufe der Rückstände vorausgeht.

4. Verfahren zur Extraktion von 2-Phenylethanol gemäß Anspruch 3, in dem die Reinigung der Rückstände durch tangentiale Mikrofiltration erfolgt.

5. Verfahren zur Extraktion von 2-Phenylethanol gemäß Anspruch 3 oder 4, in dem die Extraktion des Phenylethanols mittels einer Adsorptionssäule erfolgt.

6. Verfahren zur Extraktion von 2-Phenylethanol gemäß Anspruch 5, in dem der 2-Phenylethanol anschließend durch Elution der Säule mit einem Alkohol oder anderen organischen Lösungsmitteln gewonnen wird.

7. Verfahren zur Extraktion von 2-Phenylethanol gemäß Anspruch 3 oder 4, in dem die Extraktion des 2-Phenylethanols durch Umkehrosmose bewirkt wird.

8. Verfahren zur Extraktion von 2-Phenylethanol gemäß Anspruch 7, in dem der 2-Phenylethanol, welcher in dem durch Umkehrosmose erhaltenen Retentat enthalten ist, anschließend mit Hilfe eines Lösungsmittels gewonnen wird.

## Claims

1. Method for extracting 2-phenylethanol, in which the 2-phenylethanol is extracted from the distillation residues obtained during the production of alcohol.

2. Method for extracting 2-phenylethanol according to Claim 1, in which the 2-phenylethanol is extracted from the second-distillation residues obtained during the production of alcohol by double-distillation.

3. Method for extracting 2-phenylethanol according to Claim 1 or 2, in which extraction of the 2-phenylethanol is preceded by a step of clarification of the residues.

4. Method for extracting 2-phenylethanol according to Claim 3, in which the residues are clarified by tangential microfiltration.

5. Method for extracting 2-phenylethanol according to Claim 3 or 4, in which the phenylethanol is extracted by means of an adsorbent column.

6. Method for extracting 2-phenylethanol according to Claim 5, in which the 2-phenylethanol is then recovered by elution of the column with an alcohol or any other organic solvent.

7. Method for extracting 2-phenylethanol according to Claim 3 or 4, in which the 2-phenylethanol is extracted by reverse osmosis.

8. Method for extracting 2-phenylethanol according to Claim 7, in which the 2-phenylethanol contained in the retained material obtained by reverse osmosis is then recovered using a solvent.
